# EUROPEAN PATENT SPECIFICATION

(11) **EP 1 042 016 B1**
(45) Date of publication and mention of the grant of the patent: **12.03.2003**
(21) Application number: 98962035.6
(22) Date of filing: 09.12.1998
(51) Int. Cl.: A61L 15/46, A61F 13/15

(54) **ODOUR CONTROL PARTICLES HAVING A LIQUID ACTIVATED PARTICLE SEPARATION MEANS**
GERUCHSKONTROLLTEILCHEN MIT FLÜSSIGKEITSAKTIVIERTEM TEILCHENTRENNUNGSMITTEL
PARTICULES DESODORISANTES COMPORTANT UN ELEMENT DE SEPARATION DE PARTICULES A ACTIVATION LIQUIDE

(30) Priority: 18.12.1997 EP 97122345
(43) Date of publication of application: 11.10.2000
(73) Proprietor: THE PROCTER & GAMBLE COMPANY, Cincinnati, Ohio 45202 (US)
(72) Inventor: CARLUCCI, Giovanni, I-66100 Chieti (IT); GAGLIARDI, Ivano, I-65123 Pescara (IT); DIVO, Michael, D-61381 Friedrichsdorf (DE); FEBO, Pietro, I-65126 Pescara (IT)
(74) Representative: Kohol, Sonia
(86) International application number: US9826168
(87) International publication number: WO99030752

(56) References cited:
- EP-A- 0 348 978
- EP-A- 0 392 528

## Description

### Background of the Invention

The present invention relates to odour controlling particles which are useful for absorbent articles in particular sanitary napkins and panty liners. Odour control particles are provided to combat a broad range of odourous compounds and are provided in a particulate and are provided with separating means which are activated on contact with liquid discharge causing the particle to separate into the individual components and thereby increasing the effect of the odour control benefit delivered.

### Field of the Invention

Whilst the primary focus of absorbent articles remains the ability of these articles to absorb and retain fluids, another important area of development in this field is the control of odourous compounds contained within the absorbed fluids or their degradation products. There are a wide range of compounds which may be present in an absorbent article during use which result in the formation of malodourous. These compounds include fatty acids, ammonia, amines, sulphur containing compounds and ketones and aldehydes.

The art is replete with descriptions of various odour controlling agents for use in absorbent articles in order to address the problem of malodour formation. These agents can typically be classified according to the type of odour the agent is intended to combat. Odours may be classified as being acidic, basic or neutral. Acidic odour controlling agents have a pH greater than 7 and typically include inorganic carbonates, bicarbonates, phosphates and sulphates. Basic odour controlling agents have a pH of less than 7 and include compounds such as citric acid, boric acid and maleic acid.

The most commonly utilised odour controlling agents are the neutral odour control agents which have a pH of approximately 7. Examples of these known types of compounds include activated carbons, clays, zeolites, silicas, starches and certain combinations thereof. Examples of such agents are described in EPO 348 978 which discloses an absorbent article comprising an odour control system wherein the neutral odour controlling particles are selected from carbon, clays, silicas, zeolites and molecular sieves. Also in EPO 510 619 which relates to an absorbent article comprising odour control complex including a combination of at least 2 agents selected from a group including zeolites and silica gels. Similarly, WO 91/11977 and WO 91/12030 disclose certain combinations of zeolites and absorbent gelling materials.

However, many of these neutral odour control agents and combinations thereof have certain associated disadvantages therewith. For example silica and silica molecular sieves are considered expensive odour control agent components. On the other hand activated carbon which has been noted in the art as providing particularly effective odour control, is not favoured due to its black appearance, which is considered unacceptable by consumers. Zeolites which unlike carbon do not have a negative aesthetic profile, are not considered to deliver effective odour control over a broad range of odour types. Moreover, another disadvantage is that the more effective types of odour control zeolites, the so-called intermediate and high ratio SiO₂/AlO₂ zeolites are also particularly expensive.

Another particular problem especially acute for multi-component odour control systems, particularly those comprising zeolite is that it is essential in such systems that the components are sufficiently blended prior to their incorporation into the absorbent article. This is in order that the odour control system can be satisfactorily homogeneously incorporated within the absorbent article at the desired location and thereby deliver the maximum odour control benefit.

Furthermore, yet another problem related to the satisfactory incorporation of many of the odour control materials, particularly zeolites within absorbent articles is concerned with the particles size of the odour control materials. in order to be readily and homogeneously incorporated into the absorbent articles without the formation of dust. The formation of dust is considered as a safety hazard and also results in the reduction of the quality of the laminate structure in the odour control particles are typically incorporated by deactivating the adhesive and hindering bonding and thereby allowing the particles to escape from the laminate and the article. Hence it is desirable that the odour control materials should preferably be provided such that they have a minimum particle size. However, in order to achieve this the use of specific manufacturing or processing techniques are often required.

In order to address the problem of dust formation, it has been suggested to adhere the particulate odour controlling agents to the surface of larger particles of absorbent gelling materials utilizing moisture. The resulting particles are less dusty, easier to handle and exhibit improved odour control. However, it has also been observed that such absorbent gelling material particles can only be loaded with up to about 10% by weight of odour control agent using water.

Attempts to increase the amount of odour controlling agents in such particles comprising absorbent gelling materials as for example described in WO 91/12029 have resided in the utilization of a binder material within the particle. Typically, this can be achieved by using a fluidized bed apparatus known as agglomeration. However, a problem related to using binder materials within odour control particles is that the binders may at least partially physically inactivate the odour control active materials. This may occur by physical covering or blocking of the pore sites of the odour control active materials, thereby rendering the sites inaccessible to the odourous compounds or alternatively, the binder materials may chemically inactive the odour control active materials. Moreover, whilst the amount of odour control active material that can be incorporated within the particle may be increased compared to the process of using moisture as a binder, the particles typically require a binder content of about 10% by weight which in order to form the particles and hence which cannot be occupied by odour control active materials.

Yet another problem with the incorporation of binder materials in odour control particles is that depending on the type of binder and the binding strength of the binder, the particles will retain their integrity even after contact with the liquid discharge. Thus whilst it is highly desirable from a manufacturing standpoint to incorporate agglomerated odour control particles within absorbent articles from a performance point of view this is highly undesirable as a large proportion of the odour control active material as a consequence will not contact the liquid discharge and hence remain un-used.

Hence there exists a need to provide odour control particles which retain their structural integrity such that they can be easily and readily handled and positioned in the desired location of the absorbent articles. Furthermore, there is also a need to provide odour control particles which separate out into the individual odour control active material components during use of the absorbent article.

The present invention provides a means to meet these objectives by providing odour control particles comprising at least three odour control active materials and which further comprise particle separation means which become activated upon contact with liquid.

### Summary of the Invention

The present invention hence relates to odour control particles which find application in absorbent articles such as sanitary napkins, diapers, incontinence products and perspiration pads. The odour control particles of the present invention comprise at least three odour control active materials and an effective amount of a liquid activated separation means. The odour control particles of the present invention offer the advantage that particles can be provided having the desired particles size in order to provide improved handling during manufacture, and which also comprise liquid activated separation means such that the particle will separate into its individual components upon contact with the liquid discharge and thus provide improved odour control performance.

### Detailed Description of the Invention

The present is thus related to the provision of odour control particles for use in absorbent articles which comprises at least three odour control actives. According to the present invention the term particle as used herein encompasses particulates, granulates, flakes, noodles and extrudates.

According to the present invention the odour control particles comprise at least three odour control active materials known in the art for that purpose. Suitable odour control active materials for use herein include zeolites, silica, carbon, chelants such as for example ethylene diaminetetraacetic acid, cyclodextrin, absorbent gelling materials (AGM), antimicrobial agents, perfuming ingredients, masking agents, and mixtures thereof. Particularly preferred are zeolites, silica, AGM, chelants and mixtures thereof. Most preferred are mixtures of AGM, silica and zeolite which are preferably present at a weight ratio of from 1:5:1 to 1:1:5, preferably from 1:3:1 to 1:1:3 and most preferably from 1:1:1 to 1:1.5:1.5. However any combination of known odour control actives can be selected by the skilled man depending on the odours which the odour control active materials should combat.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred are zeolite A, zeolite Y or mixtures thereof. Preferred cations may be selected from group I and II metals and may also include heavy metals such as for example zinc, silver and copper. According to the present invention the zeolite is preferably hydrophobic. This is typically achieved by increasing the molar ratio of the SiO₂ to AlO₂ content such that the ratio of x to y is at least 1, preferably from 1 to 500, most preferably from 1 to 6.

According to the present invention the compacted odour control particles further comprise an effective amount of a liquid activated particle separation means. The incorporation of such a separating means within the particle allows the particle to be introduced into the absorbent article homogeneously and without the problem of dust formation and once the absorbent article is in use and the particles come into contact with the liquid discharge the particles separate out, preferably into the individual components. This thereby ensures that all of the odour control actives come into contact with liquid discharge and also provides a larger surface area for liquid contact. In this manner the odour control benefit provided by the particles is increased.

According to the present invention the term liquid activated separation means as used herein refers to a material which when incorporated in the compacted odour control particles and contacted dropwise with 1 ml of distilled water at 25° C, particle separation as defined herein occurs within 10 minutes, preferably within 5 minutes, more preferably within 1 minutes and most preferably within 30 seconds, as an average of 10 tests.

The term particle separation as used herein, refers to the odour control particles of the present invention no longer exhibit structural integrity and at least one of the odour control active materials is no longer associated with the particle.

The effective amount of liquid activated particle separation means which is incorporated within the odour control particle can be readily determined by the skilled person and is selected such that there is a sufficient amount in order to ensure particle separation as described above. The amount required is in principle dependent upon a number of factors which include, the nature and number of the different types of the odour control active materials contained in the particle, the amount and density of the particles, the process utilised to manufacture the odour control particles for example dry pressure compaction or agglomeration and the strength of the separation means itself. Typically, the weight ratio of the separation means to the total weight of the odor control active materials is from 1:10 to 10:1 preferably from 1:7 to 7:1, more preferably 1:5 to 5:1, most preferably 1:2 to 2:1.

According to the present invention any material which causes particle separation upon liquid activation as defined herein above may be utilized. Typically, the separation means fall into two categories; physical particle separation means and chemical particle separation means. Separation by physical separation occurs when the separation means physically interacts with liquid to cause separation. This may occurs via mechanisms such as swellage, shrinkage, absorption or solubilization. Separation by chemical interaction with the liquid occurs when the separating means undergoes a chemical reaction on contact with liquid producing resultant products and often by-products. The products of such chemical reactions may be in the same physical state as the starting material or preferably are in a different physical state, as this accelerates the particle separation. Most preferably at least one of the products of such a chemical separation is in the gaseous state.

Examples of suitable physical separating means for use herein include polyvinyl alcohols preferably having a hydrolysis level of at least 85 %, more preferably at least 95 %, most preferably at least 98 %. The polyvinyl alcohols are prepared by hydrolysis of polyvinyl acetate and thus the term hydrolysis level as used herein thus refers to the percent, in moles, of substitution of the acetate groups by hydroxyl groups.

Other suitable materials for use herein include polymers such as crosslinked acrylic acid and its copolymers, especially in the form of full or partial salts of alkaline metals, alkaline-earth metals as well as aluminium, zinc and iron salts, as well as such as poly(methacrylic acid), polyacrylamide, poly(N,N-dimethylacrylamide), and polymethylacrylamide;
crosslinked poly-vinyl-pyrrolidone; copolymers of poly-vinyl-pyrrolidone, particularly those containing vinyl acetate; polymethyl-, polyethyl- or polybutyl-vinyl ethers as well as the copolymers derived from vinyl ethers as, for example, the copolymers of methyl-vinyl-ether and maleic anhydride or maleic acid;
polyethylene oxide having molecular weights from about 100,000 to 8,000,000 Daltons; and
polyethylenimine; polyacrolein; styrene-maleic anhydride copolymers; ethylene-maleic anhydride copolymers; polydimethylaminoethyl methacrylate; polyalkylene polyamines; poly(vinylbenzyl-trimethylammonium chloride) as well similar quaternary ammonium polymers; poly(maleic anhydride); lower molecular weight phenol-formaldehyde resins; lower molecular weight ureaformaldehyde resins; and
water soluble salts such as sodium and potassium chloride and sulphate and mixtures thereof.

Suitable chemical separating means for use herein include sodium and potassium bicarbonate, citrate, tartrate, tartaric acid, citric acid and mixtures thereof. In addition, the separating means may also comprise a mixture of physical and chemical separation means. In the most preferred embodiment, the particle separation means in addition to providing particle separation, also provides as odour control benefit, such as AGM.

In addition to the odour control active materials and the liquid separation means of the present invention, the odour control particles may also comprise optional components such as perfuming ingredients or masking agents known to the skilled person in the art.

According to the present invention the odour control particles may be prepared using any method known in the art such as by agglomeration, spray drying techniques, fluid bed coating or high pressure compaction using press rolls which are described more fully herein after. Particularly preferred methods are agglomeration and high pressure compaction.

In order to prepare the odour control particles utilizing agglomeration techniques it is also particularly preferable to additionally employ a binder material, although in certain circumstances the liquid separation means may also provide binder capability and hence not be required. Any binder materials used in agglomeration or spray drying techniques may be used herein, for example starches, cellulose, gums, anionic and nonionic surfactants such as PEG, fatty acids, fatty alcohols, ethoxylates or any mixtures thereof.

Preferred binder materials are well known in commerce under various trade names such as GELFORM, PURAGEL, LAVERAL, MALTRIN and METHOCEL. In general these binders are soluble or dispersible in water or body fluids such as blood and urine. Chemically such preferred binders comprise various starch, cellulose, modified starch, modified cellulose, gum acacia, gum arabic, soluble gelatine materials or mixtures thereof. Carboxymethylcellulose and hydroxypropylcellulose are more preferred binders for use herein. Typically, the above described binder will comprise at least 1 %, preferably from 3 % to 15 %, most preferably from 4 % to 10 % by weight of the final odour control particles. The amount of binder used can be readily determined by the skilled man and will depend on the binder material used and the desired particle size of the odour control system.

Accordingly, odour control particles prepared utilizing agglomeration techniques comprise the process steps of mixing, transferring to a high speed mixer, agglomerating and drying. It will be understood that any convenient order of the process steps listed above can be contemplated. Also it may be possible and even advantageous to carry out two or more of the above operations in a single piece of process equipment. Each of these operations will now be described in more detail.
1. Making a Paste Premix: The paste premix may be prepared by any method which is known to the man skilled in the art. In the present invention the liquid separation means and binder material are mixed with water and the optional ingredients to form a paste. This may be achieved in any convenient piece of mixing equipment, and may be carried out using any order of addition of the separate or pre-mixed components.
2. & 3. Fine Dispersion Mixing and Granulation: For mixing/ agglomeration of the odour control active materials with the paste any of a number of mixers/agglomerators can be used, particularly high and middle shear mixers. In one preferred embodiment, the process of the invention is continuously carried out. Especially preferred are mixers of the Fukae^{R} FS-G series manufactured by Fukae Powtech Kogyo Co., Japan; this apparatus is essentially in the form of a bowl-shaped vessel accessible via a top port, provided near its base with a stirrer having a substantially vertical axis, and a cutter positioned on a side wall. The stirrer and cutter may be operated independently of one another and at separately variable speeds. The vessel can be fitted with a cooling jacket or, if necessary, a cryogenic unit.
   Other similar mixers found to be suitable for use in the process of the invention include Diosna^{R} V series ex Dierks & Söhne, Germany; and the Pharma Matrix^{R} ex T K Fielder Ltd., England. Other mixers believed to be suitable for use in the process of the invention are the Fuji^{R} VG-C series ex Fuji Sangyo Co., Japan; and the Roto^{R} ex Zanchetta & Co srl, Italy.
   Other preferred suitable equipment can include Eirich^{R}, series RV, manufactured by Gustau Eirich Hardheim, Germany; Lödige^{R}, series FM for batch mixing, series Baud CB/KM for continuous mixing/agglomeration, manufactured by Lödige Machinenbau GmbH, Paderborn Germany; Drais^{R} T160 series, manufactured by Drais Werke GmbH, Mannheim Germany; and Winkworth^{R} RT 25 series, manufactured by Winkworth Machinery Ltd., Berkshire, England.
   The paste can be introduced into the mixer at an initial temperature between its softening point (generally in the range of 40 - 60° C) and its degradation point (depending on the chemical nature of the paste). High temperatures reduce viscosity simplifying the pumping of the paste but result in lower active agglomerates.
   The introduction of the paste into the mixer can be done in many ways, from simply pouring to high pressure pumping through small holes at the end of the pipe, before the entrance to the mixer. The method utilised depending principally on the binder material used. If necessary, extrusion of the paste and/or pumping pressures prior to the entrance in the mixer may be utilised.
4. Drying: It is also within the scope of the present invention that the resulting granules may be dried, for example, using a fluidized bed or cooled and/or dusted with a suitable surface coating agent.

According to the present invention the odour control particles may also be prepared using spray drying methods known in the art. Using such a process a mixture of the odour control active materials, liquid separation means and a binder is mixed in a vessel with stirring. Water is added along with optional viscosity modifying agents. The pH is adjusted to be greater than 10 and additional water is added in order to provide the mixture with the desired flow characteristics. The mixture is stirred for up to 1 hour. The mixture is then transferred to the top of a spray drying tower unit. The inlet temperature and the resonance time is varied to produce the desired particle size and moisture content. The resulting spray dried powder is collected at the base of the tower.

Fluidized bed coating techniques may also be utilized to provide the odour control particles of the present invention. Binder is dissolved in deionised water. The odour control active materials and liquid separation means are added to this solution and a high shear mixer is used to create a dispersion.

Particle separation means are placed in a fluid bed coater. The means are fluidised in the bed at an air flow and temperature readily selected by the skilled person in the field. The binder, liquid separation means and odour control active material coating solution is sprayed from the spray nozzle at the required flow rate and air temperature.

Another method which may be usefully applied to manufacture the odour control particles of the present invention is high pressure compaction. The method involves high pressure compaction of odour control active material, using press rolls, as described more fully in the examples below. This method provides an improved alternative to conventional wet agglomeration techniques which require a binder material.

In principle the pressure compaction methods involves the following steps. Firstly, the odour control active materials and liquid separation means to be used are combined to form a homogeneous mixture. The mixture of actives are then predensified in a predensifier (vertical screw) to remove air and improve the densification of the actives and to obtain the required particle size. The mixture is then compacted with a compactor (Type: series L 200/50 P smooth rolls) by pressure selected from 10 to 100 KN/cm² to form tablets. The tablets then finally ground to form odour control particles of the required size. As a result of compaction the density of the resultant odour control particles increases by at least 40 %, preferably 100 %, more preferably 150 % and most preferably by at least 200 % of the density of the mixture of odour control actives prior to the compaction process.

According to the present invention the odour control particles may have any particle size as desired and it can thus be selected according to the end use of the particles envisioned. Typically for applications in absorbent articles such as sanitary napkins, the particles should preferably have a mean particle size such that no more than 5 % of the particles are greater than 1.7mm in diameter and not more than 5 % are less than 0.15mm in diameter. Preferably, the mean particle size is from 0.1mm to 2.0mm, preferably from 0.2mm to 0.7mm, most preferably from 0.3mm to 0.5mm. The particle size can be readily determined by sieve analysis.

The odour control particles of the present invention find particular utility within absorbent articles. Particularly, suitable absorbent articles include sanitary napkins, panty liners, diapers, incontinence products and perspiration pads. In particular, the present invention finds application in sanitary napkins and panty liners.

### Absorbent article

In order to more fully assess the utility of the process of the present invention a description of a typical disposable absorbent article follows.

Typically absorbent articles comprise a liquid permeable topsheet, a liquid impervious backsheet and an absorbent structure positioned in-between the topsheet and backsheet.

### The topsheet

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure. The topsheet can extend and form part or all of the preferred side flaps, side wrapping elements or wings.

When referring to the topsheet a multi layer structure or a mono layer structure is contemplated. The hybrid topsheet mentioned above is such a multi layer design but other multi layer topsheets such as primary and secondary topsheet designs are also considered.

### Absorbent core

According to the present invention the absorbent cores suitable for use in herein may be selected from any of the absorbent cores or core system known in the art. As used herein the term absorbent core refers to any material or multiple material layers whose primary function is to absorb, store and distribute fluid.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in addition to the absorbent gelling material that may be provided within the odour control particles of the present invention, usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### Backsheet

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance.

According to a preferred embodiment of the present invention the backsheet of the absorbent article is moisture vapour permeable and thus comprises at least one gas permeable layer. Suitable gas permeable layers include 2 dimensional, planar micro and macro-porous films, macroscopically expanded films, formed apertured films and monolithic films. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional planar layers of the backsheet may be made of any material known in the art, but are preferably manufactured from commonly available polymeric materials. Suitable materials are for example Gortex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core.

Suitable macroscopically expanded films for use herein include films as described in for example in US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland such as Pebax™, available from Elf Atochem (France) and Estane™, available from BF Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven. The most preferred breathable backsheet component comprises a microporous film and an apertured formed film or a microporous film and a hydrophobic nonwoven or woven textile.

In addition to the components described herein above, the absorbent article may also comprise all those features and parts which are typical for products in the context of their intended use such as wings and side flaps, undergarment adhesive means, release paper, wrapping elements, fastening means and the like.

According to the present invention the absorbent article is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof.

The odour control particles may be incorporated into the absorbent article by any of the methods disclosed in the art,. Typically the odour control particles are positioned within the absorbent core structure, for example the particles may be layered on the core of the absorbent article or mixed within the fibres of the absorbent core. The odour control system is preferably incorporated between two layers of air laid cellulose tissue. Optionally the particles may be bonded between two cellulose tissue layers with, for example, a hot melt adhesive or any suitable bonding system. The adhesive is preferably also placed on the edges of the laminate to ensure that the edges of the laminate adhere to one another so that any loose odour control particles not attached to the laminate cannot fall out of the laminate. More preferably the odour control particles is incorporated in a layered structure in accordance with the disclosure of WO 94/01069.

According to the present invention the amount of the odour control particles which may be used in the absorbent article can be readily determined by the skilled person bearing in mind the type of absorbent articles and the dimensions of the absorbent article. For example the absorbent article may comprise from 0.2g to 15g, preferably from 0.5g to 5g, most preferably from 1.5g to 2.5g of said odour control system.

### Examples

### Example 1: Dry pressure compacted odour control particles:

0.74g of zealot (zeolite Wessalith P, available from Degussa) having a particle size of 0.0035mm, 1.0g of silica (silica syloblanc 82, available from Grace) having a particle size of 0.009mm, 0.5g of EDTA (available from BASF, Germany) and 0.74g of AGM (AGM -Aquaic L74, available from Shokubay) having a particle size of 0.45mm are mixed together. The mixture is then precompressed and air is eliminated therefrom. The mixture is then compacted using a high pressure compactor roll press (available from Hosakawa Bepax GmbH, Germany) to produce a tablet. The tablets are then pre-broken into chips or flakes which are then ground to form the odour control particles of the desired size. The particles are then screened by (passing through a sieve) and the oversized and fine particles are recycled for reprocessing.

### Example 2: Agglomerated odour control particles

This example is identical to example 1 in terms of the odour control materials but further comprises 5% by weight of carboxymethylcellulose as a binder material. The particles are prepared utilising the agglomeration techniques described herein above.

## Claims

1. Odour control particles for absorbent articles, said odour control particles comprising at least three odour control active materials and an effective amount of a liquid activated particle separating means.

2. Odour control particles according to claim 1, wherein said odour control active materials are selected from zeolites, silica, absorbent gelling material, chelants, active carbon and mixtures thereof.

3. Odour control particles according to claim 2, wherein said odour control active materials are zeolite, silica and absorbent gelling material, preferably at a weight ratio of 1:5:1 to 5:1:1.

4. Odour control particles according to any one of the preceding claim wherein said odour control particles comprise said separating means at a weight ratio to the total odour control actives of from 1:10 to 10:1.

5. Odour control particles according to any one of the preceding claims wherein said liquid activated particle separating means is a physical separating means.

6. Odour control particles according to claim 5, wherein said physical separating means is a material selected from absorbent gelling material, polyvinyl alcohol, sodium chloride or mixtures thereof.

7. Odour control particles according to any one of the preceding claims, wherein said odour control particles further comprises at least 1 % by weight of a binder.

8. Odour control particles according to any one of the preceding claims, wherein said odour control particles are compacted or agglomerated

9. An absorbent article comprising from 0.2g to 15g of said odour control particles, preferably from 0.5g to 5g according to any one of the preceding claims.

10. An absorbent article according to claim 9, wherein said particles have an average particle size of from 0.1 mm to 2.0mm.

11. An absorbent article according to claim 9, comprising a topsheet an absorbent core and a backsheet, wherein said core is intermediate said backsheet and said topsheet and wherein said backsheet is moisture vapour permeable.

## Patentansprüche

1. Geruchsbekämpfungsteilchen für absorbierende Artikel, wobei diese Geruchsbekämpfungsteilchen mindestens drei für die Geruchsbekämpfung aktive Materialien und eine wirksame Menge eines durch Flüssigkeit aktivierten Teilchentrennungsmittels umfassen.

2. Geruchsbekämpfungsteilchen nach Anspruch 1, wobei die für die Geruchsbekämpfung aktiven Materialien aus Zeolithen, Silica, einem absorbierenden Geliermaterial, Chelaten, Aktivkohle und Mischungen daraus ausgewählt werden.

3. Geruchsbekämpfungsteilchen nach Anspruch 2, wobei die für die Geruchsbekämpfung aktiven Materialien Zeolith, Silica und absorbierendes Geliermaterial sind, die vorzugsweise in einem Gewichtsverhältnis von 1:5:1 bis 5:1:1 vorliegen.

4. Geruchsbekämpfungsteilchen nach irgend einem der vorhergehenden Ansprüche, wobei die Geruchsbekämpfungsteilchen ein Trennungsmittel in einem Gewichtsverhältnis zu den gesamten für die Geruchsbekämpfung aktiven Materialien im Verhältnis von 1:10 bis 10:1 umfassen.

5. Geruchsbekämpfungsteilchen nach einem der vorhergehenden Ansprüche, wobei das durch Flüssigkeit aktivierte Teilchentrennungsmittel ein physikalisches Trennungsmittel ist.

6. Geruchsbekämpfungsteilchen nach Anspruch 5, wobei das physikalische Trennungsmittel ein Material ist, das aus absorbierendem Geliermaterial, Polyvinylalkohol, Natriumchlorid oder Mischungen daraus ausgewählt wird.

7. Geruchsbekämpfungsteilchen nach einem der vorhergehenden Ansprüche, wobei die Geruchsbekämpfungsteilchen weiter mindestens 1 Gewichtsprozent eines Bindemittels umfassen.

8. Geruchsbekämpfungsteilchen nach einem der vorhergehenden Ansprüche, wobei die Geruchsbekämpfungsteilchen verfestigt oder agglomeriert sind.

9. Absorbierender Artikel, der 0,2 g bis 15 g, vorzugsweise 0,5 g bis 5 g der Geruchsbekämpfungsteilchen gemäß einem der vorherigen Ansprüche umfasst.

10. Absorbierender Artikel nach Anspruch 9, wobei die Teilchen eine mittlere Teilchengröße von 0,1 mm bis 2,0 mm aufweisen.

11. Absorbierender Artikel nach Anspruch 9, der eine Oberschicht, einen absorbierenden Kern und eine Unterschicht umfasst, wobei der Kern sich zwischen der Unterschicht und der Oberschicht befindet, und wobei die Unterschicht für Wasserdampf durchlässig ist.

## Revendications

1. Particules de limitation d'odeur pour articles absorbants, lesdites particules de limitation d'odeur comprenant au moins trois matériaux actifs de limitation d'odeur et une quantité efficace d'un moyen de séparation de particules liquide activé.

2. Particules de limitation d'odeur selon la revendication 1, dans lesquelles lesdits matériaux actifs de limitation d'odeur sont choisis parmi les zéolites, la silice, les matériaux absorbants gélifiants, les chélatants, le charbon actif et leurs mélanges.

3. Particules de limitation d'odeur selon la revendication 2, dans lesquelles lesdits matériaux actifs de limitation d'odeur sont composés de zéolite, silice, matériau absorbant gélifiant, de préférence, dans un rapport pondéral de 1:5:1 à 5:1:1.

4. Particules de limitation d'odeur selon l'une quelconque des revendications précédentes, dans lesquelles lesdites particules de limitation d'odeur comprennent ledit moyen de séparation dans un rapport pondéral, par rapport au poids total des matériaux actifs de limitation d'odeur, compris entre 1:10 et 10:1.

5. Particules de limitation d'odeur selon l'une quelconque des revendications précédentes, dans lesquelles ledit moyen de séparation de particules liquide activé est un moyen de séparation physique.

6. Particules de limitation d'odeur selon la revendication 5, dans lesquelles ledit moyen de séparation physique est un matériau choisi parmi les matériaux absorbants gélifiants, l'alcool polyvinylique, le chlorure de sodium ou leurs mélanges.

7. Particules de limitation d'odeur selon l'une quelconque des revendications précédentes, dans lesquelles lesdites particules de limitation d'odeur comprennent, en outre, au moins 1 % en poids de liant.

8. Particules de limitation d'odeur selon l'une quelconque des revendications précédentes, dans lesquelles lesdites particules de limitation d'odeur sont compactées ou agglomérées.

9. Article absorbant comprenant de 0,2 g à 15 g desdites particules de limitation d'odeur, de préférence, de 0,5 g à 5 g, selon l'une quelconque des revendications précédentes.

10. Article absorbant selon la revendication 9, dans lequel lesdites particules ont une taille de particule moyenne comprise entre 0,1 mm et 2,0 mm.

11. Article absorbant selon la revendication 9, comprenant une feuille de dessus, une âme absorbante et une feuille de fond, dans lequel ladite âme est placée entre ladite feuille de fond et ladite feuille de dessus, et dans laquelle la feuille de fond est perméable à la vapeur humide.
